Europäisches Patentamt
European Patent Office
Office européen des brevets

(19)

(11) Publication number: **0 186 452**
**A2**

(12) **EUROPEAN PATENT APPLICATION**

(21) Application number: 85309289.8

(22) Date of filing: 19.12.85

(51) Int. Cl.[4]: C 07 F 7/18
A 61 K 31/695

(30) Priority: 28.12.84 JP 274465/84

(43) Date of publication of application: 02.07.86 Bulletin 86/27

(84) Designated Contracting States: CH DE FR GB IT LI NL SE

(71) Applicant: SANWA KAGAKU KENKYUSHO CO., LTD.
No. 35, Higashi-sotobori-cho
Higashi-ku Nagoya-shi Aichi-ken(JP)

(72) Inventor: Toyoshima, Shigeshi
3-14-22, Funabashi Setagaya-ku
Tokyo(JP)

(72) Inventor: Kurono, Masayasu
1-7-17, Kakeage Minami-ku
Nagoya-shi Aichi-ken(JP)

(72) Inventor: Unno, Ryoichi
1-3, Hikarigaoka Chikusa-ku
Nagoya-shi Aichi-ken(JP)

(72) Inventor: Kimura, Hiromoto
10-197-3, Jojo-cho
Kasugai-shi Aichi-ken(JP)

(72) Inventor: Ito, Koichi
Shin-Etsu Chem. Co., Ltd. 6-1, Ohtemachi 2-chome
Chiyoda-ku Tokyo(JP)

(72) Inventor: Ishihara, Toshinobu Shin-Etsu Chem.Co.,Ltd.
Gosei-Gijutsu Kenkyusho 28-1,Ohaza Nishi-fukushima
Kubiki-mura Naka-kubiki-gun Niigata-ken(JP)

(72) Inventor: Yamamoto, Akira c/o Shin-Etsu Chem.Co.,Ltd.
Gosei-gijutsu Kenkyusho 28-1,Ohaza Nishi-fukushima
Kubiki-mura Naka-kubiki-gun Niigata-ken(JP)

(74) Representative: Diamond, Bryan Clive et al,
Gee & Co. Chancery House Chancery Lane
London WC2A 1QU(GB)

(54) **Organosilicon compounds process for their production and pharmaceutical compositions thereof.**

(57) Organosilicon compounds are represented by the formula:

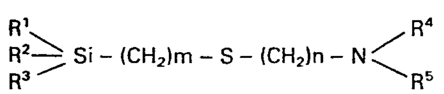

(I)

wherein $R^1$, $R^2$ and $R^3$ are each alkyl, cycloalkyl, alkoxy, phenyl, substituted phenyl, alkylcarbonyloxy or trialkylsilyloxy, $R^4$ and $R^5$ are each hydrogen or alkyl and *m* and *n* are integers, and non-toxic salts thereof.

Suitable salts are the terephalate and maleate.

The compounds are synthesised by reacting $R^1$, $R^2$, $R^3$ Si $(CH_2)_r$ $-CH=CH_2$ + $HS-(CH_2)n-NR^4R^5$ where r is an integer, in a solvent with ultraviolet irradiation in the presence of a photosensitive compound, e.g. benzophenone; and optionally forming a salt.

The compounds are formed into usual pharmaceutical compositions, e.g. tablets, capsules, granules or suppositories. The compounds have good activity against mice skin cancers; the 5-fluoroacil derivatives have good activity against human colon cancer.

EP 0 186 452 A2

Croydon Printing Company Ltd.

# ORGANOSILICON COMPOUNDS, PROCESS FOR THEIR PRODUCTION AND PHARMACEUTICAL COMPOSITIONS THEREOF

The present invention relates to novel organosilicon compounds, a process for their production, and anti-tumor pharmaceutical compositions which contain as an effective component at least one of the compounds. Each of the compounds may also be used as an intermediate for synthesizing other compounds which show excellent anti-tumor activity.

As silicon-containing organocompounds having a pharmaceutical activity and more particularly anti-tumor action, so-called "Silatorane Compounds" have been known, which were developed by some of the present inventors and are disclosed in European Patent Specn. No.EP 0046242, but almost all of those silatorane compounds have a disadvantage in actual or clinical use, in view of their high toxicity.

There is a compound of 5-fluorouracil which has been clinically employed as an anti-tumor agent. This compound has an excellent anti-tumor action but in oral dosage thereof has a high toxicity in that it often causes a certain serious trouble in digestive canals, and thus this compound has exclusively been dosed by injection. In order to overcome this disadvantage, namely to make oral dosage possible, 1-(2-tetrahydrofuryl)-5-fluorouracil has ben synthesised; this compound has a lower toxicity in oral dosage, but has a disadvantage in that the anti-tumor activity thereof is also low.

An object of the invention is to provide novel organosilicon compounds, having a high anti-tumor activity and having no or little toxicity, particularly in oral dosage thereof, whereby such a conflict between the level of anti-tumor activity and safety in actual use, which has been encountered in the prior anti-tumor agents, can be overcome.

Another object of the invention is to provide such an organosilicon compound which is useful as an intermediate for preparing compounds which have a more excellent or powerful anti-tumor activity than the intermediate.

According to the invention, we provide novel organosilicon compounds represented by the general formula.

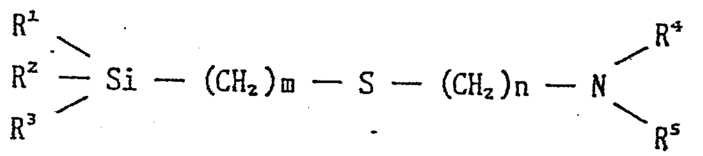

(I)

wherein $R^1$, $R^2$, and $R^3$ are each an alkyl, alkoxy, phenyl, substituted phenyl, alkylcarbonyloxy or trialkylsilyloxy group, $R^4$ and $R^5$ are a hydrogen atom or alkyl group, and _m_ and _n_ are each an integer, and non-toxic pharmaceutically acceptable salts thereof.

In the compound of Formula I, the alkyl group may be a straight-chain alkyl radical, branched-chain alkyl radical or cyclo-alkyl radical. Examples of the straight-chain alkyl radicals, having 1 to 10 carbon atoms, are methyl, ethyl, n-propyl, n-butyl, n-pentyl, n-hexyl and n-decyl radicals . Examples of the branched-chain alkyl radicals are isopropyl, isobutyl, s-butyl, t-butyl and isopentyl. Examples of the cycloalkyl radicals, having 3 or more carbons atoms, are cyclopropyl, cyclobutyl, cyclopentyl, cyclohexyl and cycloheptyl. Examples of alkoxy group are methoxy, ethoxy and 2-methoxyethoxy. Suitable substituents for the substituted phenyl radical are p-chloro, p-bromo, p-methyl and p-methoxy.

The trialkylsilyloxy group can be trimethylsilyloxy or triethyl-silyloxy.

Suitable non-toxic salts of the compounds are terephthalate and maleate.

According to a method of the invention, the compounds I and non-toxic salts canbe prepared by reacting a compound represented by the formula

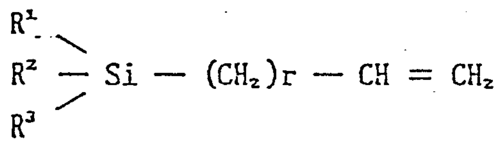

(II)

wherein $R^1$, $R^2$ and $R^3$ are as defined above, and *r* is an integer with an $\omega$-mercaptoalkylamine represented by the formula

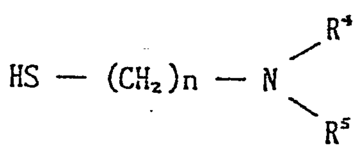

(III)

wherein $R^4$, $R^5$ and *n* are as defined above, and if necessary converting the resulting compound into the non-toxic salt in a known manner.

The reaction between the compounds II and III can be carried out by using them in an equimolar amount and under ultraviolet irradiation, in the presence of a solvent and a catalytic amount of a photosensitizer compound. As the solvent, tetrahydrofuran, dioxane, N-N-dimethylformamide, pyridine, methanol, ethanol, water or the like may be used. As the photosensitizer, benzophenone, acetophenone, acetone, benzaldehyde or the like may be employed. The reaction temperature is not within fixed limits since it depends on the nature of the raw materials and solvents, but a temperature between 10 to 60°C is preferable usually.

Each of the compounds of the Formula I can be employed as an intermediate for preparing another organosilicon compound which shows a more powerful anti-tumor activity. For this purpose, the compound I is reacted with 5-fluorouracil. The resulting 5-fluorouracil derivatives show a quite low toxicity in oral dosage thereof but excellent anti-tumor action, in comparision with the original 5-fluorouracil. Further, the derivatives show a higher anti-tumor action against colon 38 adenocarcinoma in mice, although conventional 5-fluorouracil anti-tumor agents have been considered as not effective against the cancer cell of colon-38.

The invention will now be further explained with reference to Examples and Pharmacological Test Examples.

<u>Summary of Compounds Prepared by Examples</u>

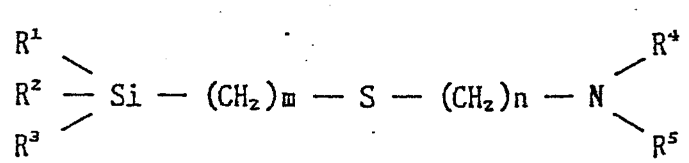

wherein the symbols have the values shown in the following table:

| Example | $R^1$ | $R^2$ | $R^3$ | $R^4$ | $R^5$ | m | n |
|---|---|---|---|---|---|---|---|
| 1 | Me | Me | n-Oct | H | H | 2 | 2 |
| 2 | Me | Me | i-Pr | H | H | 2 | 2 |
| 3 | Me | Me | n-Bu | H | H | 2 | 2 |
| 4 | Me | Ph | Ph | H | H | 2 | 2 |
| 5 | Me | Me | i-Pr | H | H | 2 | 3 |
| 6 | Me | Me | i-Pr | H | H | 3 | 2 |
| 7 | Me | Me | t-Bu | H | H | 3 | 2 |
| 8 | Me | Me | i-Pr | H | H | 3 | 3 |
| 9 | Me | Me | t-Bu | H | H | 3 | 3 |
| 10 | Me | Me | n-Bu | H | H | 3 | 3 |
| 11 | Me | Me | Ph | H | H | 3 | 3 |

Example 1

2-[[2-(Dimethyl-n-octylsilyl)ethyl]thio]ethylamine

To a 1 liter Pyrex vessel equipped with a 100W high-pressure mercury lamp fitted with a water cooled Pyrex jacket, a magnetic stirrer and a thermometer, dimethyl-n-octylvinylsilane (81.2g, 0.410 mol), 2-aminoethanethiol (30.8g, 0.400 mol) and benzophenone (2.00g, 11.0 mmol) in 300 ml of tetrahydrofuran and 20 ml of water were added.

The mixture was irradiated under nitrogen gas at 25°C.

The progress of the photolyses was judged by a disappearance of the 2-aminoethanethiol peak of gas chromatography.

The reaction mixture was fractionated to obtain the desired compound (79.2g, 72.0%).

Boiling point: 134 - 139℃/1 mmHg

Elementary analysis: $C_{14}H_{33}NSSi$

Cal. : C, 61.02; H, 12.00; N, 5.08

Found : C, 61.22; H, 11.92; N, 5.19

IR ($\nu_{max}^{neat}$ $cm^{-1}$) :

3400, 3300 ($\nu_{NH_2}$), 2950, 2875 ($\nu_{CH}$)

$^1$H-NMR ($CDCl_3$) $\delta$ :

0.00 (6H, s, $(CH_3)_2Si<$)

0.17 - 1.67 (19H, m, $CH_3(CH_2)_7SiCH_2-$)

1.43 (2H, brs, $NH_2$)

2.43 - 3.03 (6H, m, $-\underline{CH_2}S\underline{CH_2}\underline{CH_2}NH_2$)

MS (EI/DI) m/z :

275 ($M^+$), 77 (base peak)

Example 2

2-[[2-(Isopropyldimethylsilyl)ethyl]thio]ethylamine

This compound was prepared by a similar procedure as in the case of Example 1, except for the treatment with isopropyldimethylsilane (52.5g, 0.410 mol) instead of dimethyl-n-octylvinylsilane.

The reaction mixture was fractionated to obtain the desired compound (59.9g, 73.0%).

Boiling point : 105 - 110℃/1 mmHg

Elementary analysis : $C_9H_{23}NSSi$

Cal. : C, 52.62; H, 11.28; N, 6.82

Found : C, 52.75; H, 11.09; N, 6.80

IR ($\nu_{max}^{neat}$ $cm^{-1}$) :

3350, 3270 ($\nu_{NH_2}$), 2930, 2850 ($\nu_{CH}$)

$^1$H-NMR ($CDCl_3$) $\delta$ :

0.00 (6H, s, $(CH_3)_2Si<$)

0.77 - 1.17 (9H, m, $(CH_3)_2CH-SiCH_2-$)

1.57 (2H, s, $NH_2$)

2.43 - 3.10 (6H, m, $-\underline{CH_2}S\underline{CH_2}\underline{CH_2}NH_2$)

MS (EI/DI) m/z :

205 ($M^+$), 134 (base peak)

Example 3

2-[[2-(n-Butyldimethylsilyl)ethyl]thio]ethylamine

This compound was prepared by the similar procedure as in the case of Example 1, except for the use of n-butyldimethylvinylsilane (58.2g, 0.410 mol) instead of dimethyl-n-octylvinylsilane.

The reaction mixture was fractionated to obtain the desired compound (66.6g, 76.0%).

Boiling point : 118 - 124℃/1 mmHg

Elementary analysis : $C_{10}H_{25}NSSi$

Cal. : C, 54.73; H, 11.48; N, 6.38

Found : C, 54.99; H, 11.28; N, 6.30

IR ($\nu_{max}^{neat}$ $cm^{-1}$) :

3350, 3270 ($\nu_{NH_2}$), 2930, 2850 ($\nu_{CH}$)

$^1$H-NMR ($CDCl_3$) δ :

0.00 (6H, s, $(CH_3)_2Si<$)

0.77 - 1.17 (11H, m, $CH_3(CH_2)_3\geq Si-CH_2-$)

1.57 (2H, s, $NH_2$)

2.43 - 3.10 (6H, m, $-\underline{CH_2}S\underline{CH_2}\underline{CH_2}NH_2$)

MS (EI/DI) m/z :

219 ($M^+$), 204 (M-15)

Example 4

2-[[2-(Methyldiphenylsilyl)ethyl]thio]ethylamine

This compound was prepared by the similar procedure as in the case of Example 1, except for the use of methyldiphenylvinylsilane (91.8g, 0.410 mol) instead of dimethyl-n-octylvinylsilane.

The reaction mixture was fractionated to obtain the desired compound (77.1g, 64.0%).

Boiling point : 175 - 180℃/1 mmHg

Elementary analysis : $C_{17}H_{23}NSSi$

Cal. : C, 67.72; H, 7.69; N, 4.65

Found : C, 67.50; H, 7.90; N, 4.55

IR ($\nu_{max}^{neat}$ $cm^{-1}$) :

3400, 3300 ($\nu_{NH_2}$)

$^1$H-NMR ($CDCl_3$) δ :

0.65 (3H, s, $CH_3Si\leqslant$ )

0.70 - 1.03 (2H, m, $\geqslant SiCH_2$-)

1.48 (2H, s, $-NH_2$)

2.52 - 3.02 (6H, m, $-\underline{CH_2}S\underline{CH_2}\underline{CH_2}NH_2$)

7.15 - 7.75 (10H, m, Aromatic H)

MS (EI/DI) m/z :

301 ($M^+$), 77 (base peak)

Example 5

3-[[2-(Isopropyldimethylsilyl)ethyl]thio]propylamine

This compound was prepared by the similar procedure as in the case of Example 1, except for the use of isopropyldimethylvinylsilane (52.5g, 0.410 mol) and 3-aminopropanethiol (36.4g, 0.400 mol) instead of dimethyl-n-octylvinylsilane and 2-aminoethanethiol respectively.

The reaction mixture was fractionated to obtain the desired compound (47.3g, 54.0%).

Boiling point : 120 - 126℃/1 mmHg

Elementary analysis : $C_{10}H_{25}NSSi$

Cal. : C, 54.73; H, 11.48; N, 6.38

Found : C, 54.80; H, 11.49; N, 6.40

IR ($\nu_{max}^{neat}$ $cm^{-1}$) :

3380, 3300 ($\nu_{NH_2}$), 2950, 2860 ($\nu_{CH}$)

$^1H$-NMR ($CDCl_3$) $\delta$ :

0.01 (6H, s, $(CH_3)_2Si<$)
0.57 - 1.23 (9H, m, $(CH_3)_2CH-Si-CH_2-$)
1.20 (2H, br., $-NH_2$)
1.70 (2H, q, J=8.0Hz, $-SCH_2\underline{CH_2}CH_2NH_2$)
2.23 - 2.93 (6H, m, $\underline{CH_2}S\underline{CH_2}CH_2\underline{CH_2}NH_2$)

MS (EI/DI) m/z :

219 ($M^+$), 176 (base peak)

Example 6

2-[[3-(Isopropyldimethylsilyl)propyl]thio]ethylamine

This compound was prepared by the similar procedure as in the case of Example 1, except for the use of allylisopropyldimethylsilane (58.2g, 0.410 mol) instead of dimethyl-n-octylvinylsilane.

The reaction mixture was fractionated to obtain the desired compound (37.7g, 43.0%).

Boiling point : 110 - 116℃/1 mmHg

Elementary analysis : $C_{10}H_{25}NSSi$

Cal. : C, 54.73; H, 11.48; N, 6.38
Found : C, 55.00; H, 11.30; N, 6.21

IR ($\nu_{max}^{neat}$ $cm^{-1}$) :

3400, 3300 ($\nu_{NH_2}$), 2960, 2880 ($\nu_{CH}$), 1600 ($\delta_{NH_2}$)

$^1H$-NMR ($CDCl_3$) $\delta$ :

0.00 (6H, s, $(CH_3)_2Si<$)
0.50 - 0.70 (2H, m, $>SiCH_2-$)
1.90 (7H, m, $(CH_3)_2CH-Si$)
1.26 (2H, s, $-NH_2$)
1.40 - 1.90 (2H, m, $-SiCH_2\underline{CH_2}CH_2S-$)
2.10 - 2.80 (6H, m, $-\underline{CH_2}S\underline{CH_2}\underline{CH_2}NH_2$)

MS (EI/DI) m/z :

219 ($M^+$)

Boiling point : 112 - 118℃/1 mmHg

Elementary analysis : $C_{11}H_{27}NSSi$

Cal. : C, 56.58; H, 11.66; N, 6.00

Found : C, 56.69; H, 11.77; N, 5.88

IR ($\nu_{max}^{neat}$ $cm^{-1}$) :

3400, 3300 ($\nu_{NH_2}$), 2950, 2870 ($\nu_{CH}$), 1590 ($\delta_{NH_2}$)

$^1$H-NMR ($CDCl_3$) δ :

0.01 (6H, s, $(CH_3)_2Si<$)

0.30 - 0.80 (9H, m, $(CH_3)_2CH-Si-CH_2-$)

1.26 (2H, s, $-NH_2$)

1.40 - 1.90 (4H, m, $-C\underline{H_2}CH_2SCH_2C\underline{H_2}CH_2NH_2$)

2.20 - 2.90 (6H, m, $-C\underline{H_2}SC\underline{H_2}CH_2C\underline{H_2}NH_2$)

MS (EI/DI) m/z :

233 ($M^+$), 199 (base peak)

Example 9

3-{[3-(t-Butyldimethylsilyl)propyl]thio}propylamine

This compound was prepared by the similar procedure as in the case of Example 1, except for the use of allyl-t-butyldimethylsilane (64.0g, 0.410 mol) and 3-aminopropanethiol (36.4g, 0.400 mol) instead of dimethyl-n-octylvinylsilane and 2-aminoethanethiol respectively.

The reaction mixture was fractionated to obtain the desired compound (47.4g, 48.0%).

Boiling point : 131 - 134℃/1 mmHg

Elementary analysis : $C_{12}H_{29}NSSi$

Cal. : C, 58.23; H, 11.81; N, 5.66

Found : C, 58.39; H, 12.00; N, 5.48

IR ($\nu_{max}^{neat}$ $cm^{-1}$) :

3360, 3280 ($\nu_{NH_2}$), 2930, 2850 ($\nu_{CH}$), 1250 ($\nu_{C-Si}$)

$^1$H-NMR ($CDCl_3$) δ :

- 0.07 (6H, s, t-Bu($\underline{CH_3}$)$_2$Si)
0.43 - 0.78 (2H, m, t-Bu($CH_3$)$_2$Si$\underline{CH_2}$-)
0.85 (9H, s, t-Bu)
1.18 (2H, s, $NH_2$)
1.31 - 1.98 (4H, m, >Si$CH_2\underline{CH_2}CH_2SCH_2\underline{CH_2}$-)
2.38 - 2.95 (6H, m, >Si$CH_2CH_2\underline{CH_2}S\underline{CH_2}CH_2\underline{CH_2}NH_2$)

MS (EI/DI) m/z :

247 ($M^+$), 190 (base peak)

Example 10

3-[[3-(n-Butyldimethylsilyl)propyl]thio]propylamine

This compound was prepared by the similar procedure as in the case of Example 1, except for the use of allyl-n-butyldimethylsilane (64.0g, 0.410 mol) and 3-aminopropanethiol (36.4g, 0.400 mol) instead of dimethyl-n-octylvinylsilane and 2-aminoethanethiol respectively.

The reaction mixture was fractionated to obtain the desired compound (41.5g, 42.0%).

Boiling point : 133 - 135℃/2 mmHg

Elementary analysis : $C_{12}H_{29}NSSi$

Cal. : C, 58.23; H, 11.81; N, 5.66
Found : C, 58.50; H, 11.89; N, 5.60

IR ($\nu_{max}^{neat}$ $cm^{-1}$) :

3370, 3300 ($\nu_{NH_2}$), 2950, 2910 ($\nu_{CH}$), 1245 ($\nu_{C-Si}$)

$^1$H-NMR ($CDCl_3$) δ :

- 0.06 (6H, s, n-Bu($CH_3$)$_2$Si-)

0.30 - 1.98 (15H, m, n-Bu($CH_3$)$_2$Si$CH_2CH_2CH_2SCH_2CH_2$-)

1.20 (2H, s, $NH_2$)

2.37 - 2.97 (6H, m, -$CH_2SCH_2CH_2CH_2NH_2$)

MS (EI/DI) m/z :

247 ($M^+$), 190 (base peak)

Example 11

3[[3-(Dimethylphenylsilyl)propyl]thio]propylamine

This compound was prepared by the similar procedure as in the case of Example 1, except for the use of allyldimethylphenylsilane (72.2g, 0.410 mol) and 3-aminopropanethiol (36.4g, 0.400 mol) instead of dimethyl-n-octylvinylsilane and 2-aminoethanethiol respectively.

The reaction mixture was fractionated to obtain the desired compound (54.5g, 51.0%)

Boiling point : 160 - 164°C/2 mmHg

Elementary analysis : $C_{14}H_{25}NSSi$

Cal. : C, 62.86; H, 9.42; N, 5.24

Found : C, 63.00; H, 9.64; N, 5.10

IR ($\nu_{max}^{neat}$ $cm^{-1}$) :

3370, 3300 ($\nu_{NH_2}$), 3060, 3050, 3020, 2920, 2860 ($\nu_{CH}$)

$^1$H-NMR ($CDCl_3$) δ :

0.25 (6H, s, Ph($CH_3$)$_2$Si-)

0.67 - 1.02 (2H, m, PH($CH_3$)$_2$Si$CH_2$-)

1.15 (2H, s, $NH_2$)

1.43 - 1.93 (4H, m, >Si$CH_2CH_2CH_2SCH_2CH_2CH_2NH_2$)

2.35 - 2.95 (6H, m, >Si$CH_2CH_2CH_2SCH_2CH_2CH_2NH_2$)

7.20 - 7.60 (5H, m, Aromatic H)

MS (EI/DI) m/z :

267 ($M^+$), 135 (base peak)

Example 12

Example 7

2[[3-(t-Butyldimethylsilyl)propyl]thio]ethylamine

This compound was prepared by the similar procedure as in the case of Example 1, except for the use of allyl-t-butyldimethylsilane (58.2g, 0.410 mol) instead of dimethyl-n-octylvinylsilane.

The reaction mixture was fractionated to obtain the desired compound (37.3g, 40.0%).

Boiling point : 114 - 118℃/1 mmHg

Elementary analysis : $C_{11}H_{27}NSSi$

Cal. : C, 56.58; H, 11.66; N, 6.00

Found : C, 56.44; H, 11.80; N, 5.75

IR ($\nu_{max}^{neat}$ $cm^{-1}$) :

3360, 3280 ($\nu_{NH_2}$), 2950, 2930, 2850 ($\nu_{CH}$)

$^1$H-NMR ($CDCl_3$) δ :

- 0.04 (6H, s, $(CH_3)_2Si<$)

0.43 - 0.80 (2H, m, $\geq SiCH_2-$)

0.88 (9H, s, $(CH_3)_3Si-$)

1.36 (2H, s, $-NH_2$)

1.33 - 1.90 (2H, m, $-SiCH_2\underline{CH_2}CH_2S-$)

2.40 - 3.03 (6H, m, $-\underline{CH_2}S\underline{CH_2}\underline{CH_2}NH_2$)

MS (EI/DI) m/z :

176 (M-t-butyl)

Example 8

3-[[3-(Isopropyldimethylsilyl)propyl]thio]propylamine

This compound was prepared by the similar procedure as in the case of Example 1, except for the use of allylisopropyldimethylsilane (58.2g, 0.410 mol) and 3-aminopropanethiol (36.4g, 0.400 mol) instead of dimethyl-n-octylvinylsilane and 2-aminoethanethiol respectively.

The reaction mixture was fractionated to obtain the desired compound (48.5g, 52.0%).

Preparation of terephthalates of amines obtained by Examples 1 to 11

To a suspension of terephthalic acid (0.1 mol) in 300 ml of ethanol, the amine (0.2 mol) obtained in one of the Examples 1 to 11 was added and stirred for 1 hour at 20℃.

The precipitate was collected by filtration and then the filtrate was concentrated to one-tenth of the original volume to obtain the crude crystalline salt.

The combined crude crystals were recrystallized to yield the terephthalate.

The physical data and recrystallization solvent for each of the terephthalates were:

2-[[2-(Dimethyl-n-octylsilyl)ethyl]thio]ethylamine 1/2 terephthalate :

Recryst. solvent; EtOH

Melting point; 223℃ (dec.)

2-[[2-(Isopropyldimethylsilyl)ethyl]thio]ethylamine 1/2 terephthalate :

Recryst. solvent; MeOH

Melting point; 226℃ (dec.)

2-[[2-(n-Butyldimethylsilyl)ethyl]thio]ethylamine 1/2 terephthalate :

Recryst. solvent; i-PrOH

Melting point; 221℃ (dec.)

2-[[2-(Methyldiphenylsilyl)ethyl]thio]ethylamine 1/2 terephthalate :

Recryst. solvent; EtOH

Melting point; 186 (dec.)

2-[[3-(Isopropyldimethylsilyl)propyl]thio]ethylamine 1/2 terephthalate :

Recryst. solvent; EtOH-EtOAc

Melting point; 229 - 234℃ (dec.)

2-[[3-(t-Butyldimethylsilyl)propyl]thio]ethylamine 1/2 terephthalate :

Recryst. solvent; EtOH

Melting point; 210℃ (dec.)

3-[[2-(Isopropyldimethylsilyl)ethyl]thio]propylamine 1/2 terephthalate :

Recryst. solvent; MeOH-EtOAc

Melting point; 214℃ (dec.)

3-[[3-(Isopropyldimethylsilyl)propyl]thio]propylamine 1/2 terephthalate :

Recryst. solvent; EtOH-EtOAc

Melting point; 193 - 194℃

3-[[3-(t-Butyldimethylsilyl)propyl]thio]propylamine 1/2 terephthalate :

Recryst. solvent; EtOH

Melting point; 230 - 232℃ (dec.)

3-[[3-(n-Butyldimethylsilyl)propyl]thio]propylamine 1/2 terephthalate :

Recryst. solvent; MeOH-EtOAc

Melting point; 242℃ (dec.)

3-[[3-(Dimethylphenylsilyl)propyl]thio]propylamine 1/2 terephthalate :

Recryst. solvent; EtOH

Melting point; 207 - 209℃ (dec.)

<u>Reference Example 1</u>

<u>1-[2-[(2-dimethyl-n-octylsilylethyl)thio]ethylcarbamoyl]-5-fluorouracil</u>

In 400 ml of dry pyridine, 13.0g (0.100 mol) of 5-fluorouracil were dissolved and 3.0g of activated carbon was added thereto. To the resulting solution kept at 5℃, 9.90g (50.0 mmol) of trichloromethyl chloroformate were added dropwise, then stirred for 1 hour at 5℃

and then an excess phosgene was removed in vacuo. To the reaction mixture, 13.8g (50.0 mmol) of 2-[[2-(dimethyl-n-octylsilyl)ethyl]-thio]ethylamine obtained through the procedure as described in Example 1 were added dropwise under argon atmosphere, and stirred for 1 hour. The reaction mixture was evaporated to dryness in vacuo and the residue was dissolved in 400 ml of ethyl acetate and 1300 ml of 3% HCl, and then the activated carbon was filtered off. The ethyl acetate layer of the filtrate was obtained, washed with 500 ml of water, dried over sodium sulfate and the solvent was evaporated in vacuo.

The resulting crude crystals were recrystallized from ethyl ether/n-hexane to obtain the desired compound (17.1g, 79.1%) having melting point of 60 - 61℃.

Reference Examples 2 to 11

Following compounds were prepared by a similar procedure as in the case of Reference Example 1.

| Ref. Ex. | Name of Compounds | m.p. (℃) |
|---|---|---|
| 2 | 1-[2-[[2-(Dimethylisopropylsilyl)ethyl]thio]-ethylcarbamoyl]-5-fluorouracil | 85 - 86 |
| 3 | 1-[2-[[2-(Dimethyl-n-butylsilyl)ethyl]thio]-ethylcarbamoyl]-5-fluorouracil | 71 - 72 |
| 4 | 1-[2-[[2-(Diphenylmethylsilyl)ethyl]thio]-ethylcarbamoyl]-5-fluorouracil | 119 - 120 |
| 5 | 1-[3-[[2-(Dimethylisopropylsilyl)ethyl]thio]-propylcarbamoyl]-5-fluorouracil | 122 - 123 |
| 6 | 1-[2-[[3-(Dimethylisopropylsilyl)propyl]thio]-ethylcarbamoyl]-5-fluorouracil | 113 - 114 |

| | | |
|---|---|---|
| 7 | 1-[2-[[3-(Dimethyl-t-butylsilyl]propyl)thio]-ethylcarbamoyl]-5-fluorouracil | 123 - 124 |
| 8 | 1-[3-[[3-(Dimethylisopropylsilyl)propyl]thio]-propylcarbamoyl]-5-fluorouracil | 101 - 102 |
| 9 | 1-[3-[[3-(t-Butyldimethylsilyl)propyl]thio]-propylcarbamoyl]-5-fluorouracil | 102 - 103 |
| 10 | 1-[3-[[3-(n-Butyldimethylsilyl)propyl]thio]-propylcarbamoyl]-5-fluorouracil | 71 - 72 |
| 11 | 1-[3-[[3-(Dimethylphenylsilyl)propyl]thio]-propylcarbamoyl]-5-fluorouracil | 51 - 52 |

Pharmacological Test Example 1

(Acute Toxicity)

Each test compound suspended in 0.1% P-1570 was orally and forcedly dosed through a gastric probe to ICR female mice (21 - 22g, age of about 5 weeks). An identification number was given to each mouse, and weighing and observation on general behaviour were done just before the dosage and each day after the same. Each dead mouse was subjected without delay to an autopsy to check the same, more particularly in detail on enterona. All living mice were killed at the 21st day from the dosage to carry out an autopsy for checking the same, more particularly on the entrails.

An $LD_{50}$ was calculated in accordance with Leed and Munch's method. Results are shown in the following Table.

| Tested Compounds | $LD_{50}$ (mg/kg) |
|---|---|
| Example | |
| 1 | > 1500 |
| 2 | 1625 |
| 3 | > 1500 |
| 4 | > 1500 |
| 5 | 750 |
| 6 | 1500 |
| 7 | > 1500 |
| 8 | > 1500 |
| 9 | > 1500 |
| 10 | > 1500 |
| 11 | > 1500 |
| Reference Example | |
| 1 | 1580 |
| 2 | 1380 |
| 3 | 1385 |
| 4 | 1400 |
| 5 | 2250 |
| 6 | 1820 |
| 7 | 1380 |
| 8 | 2120 |
| 9 | 1800 |
| 10 | 1850 |
| 11 | 1800 |

Pharmacological Test Example 2

(Anti-tumor action to B-16 melanoma)

A cancer cell of B-16 melanoma ($1 \times 10^5$ cells) was transplanted under skin of $BDF_1$ female mice (age of about 5 weeks), and each of

testing compounds was orally dosed at 1st, 5th and 9th day after the transplantation. At 21st day after the transplantation, the mice were killed to measure a weight of the formed cancerous tumor and to finally determine an inhibition ratio due to the dosage of the test compound.

Results are shown in the following Table.

| Tested Compounds | Dosage/day x 3 mg/kg | Inhibition Ratio (%) |
|---|---|---|
| Example | | |
| 2 | 250 | 23.8 |
| | 500 | 36.1 |
| 5 | 250 | 21.4 |
| | 500 | 50.6 |
| 7 | 250 | 50.0 |
| | 500 | 32.7 |
| 8 | 250 | 61.1 |
| | 500 | 46.3 |
| 9 | 250 | 57.3 |
| | 500 | 51.9 |
| 10 | 250 | 50.4 |
| | 500 | 29.8 |
| 11 | 250 | 38.7 |
| | 500 | 45.4 |

| | | |
|---|---|---|
| Reference Example 2 | 300 | 79.7 |
| 5 | 250 | 19.4 |
| | 125 | 17.6 |
| 7 | 250 | 43.1 |
| 8 | 250 | 45.7 |
| | 125 | 32.8 |
| 9 | 250 | 46.3 |
| | 125 | 55.2 |
| 10 | 250 | 88.5 |
| | 125 | 69.5 |
| 11 | 250 | 70.5 |
| | 125 | 82.2 |

Pharmacological Test Example 3

(Anti-tumor action to colon-38 carcinoma)

0.02 ml of 10(W/V) cell suspension prepared from a solid cancer cell (colon-38 carcinoma) was transplanted under skin of $BDF_1$ female mice (20 - 21g, age of about 5 weeks), and each test compound was orally dosed at 1st, 5th and 9th day after the transplantation. major and minor axes of the forming cancerous tumor were measured at 11th, 14th, 18th, 21st, 25th and 28th day after the transplantation to calcuate the volume ofthe cancerous tumor in accordance with the equation:

$$\frac{1}{2} L \times W^2$$

wherein L: major axis (mm)

W: minor axis (mm)

At 28th day from the transplantation, the mice were killed and each of the cancerous tumors was enucleated to measure its weight and to

determine an inhibition ratio in comparison with that in a non-treated control group.

Results are shown in the following Table. From the results, it can be seen that the phamacological activity on the human colon cancer of the compounds according to the invention greatly increases, when the compound is made into its 5-fluorouracil derivative.

| Tested Compounds | Dosage/day x 3 (mg/kg) | Inhibition Ratio (%) |
| --- | --- | --- |
| Examples 1 to 11 | 270 - 350 | < 20 (No noticeable effect can be recognized) |
| Reference Example | | |
| 1 | 345 | 80.0 |
| 2 | 289 | 89.8 |
| 3 | 300 | 81.2 |
| 4 | 366 | 32.2 |
| 5 | 338 | 98.4 |
| 6 | 338 | 95.9 |
| 7 | 311 | 81.0 |
| 8 | 350 | 90.3 |
| 9 | 323 | 90.1 |
| 10 | 323 | 92.3 |
| 11 | 339 | 97.9 |

Prescription Example 1 (Tablet)

Following components were mixed to prepare tablets in a conventional manner.

| | |
| --- | --- |
| Compound of Example 1 | 100 (mg) |
| Crystalline cellulose | 20 |
| Lactose | 41 |

| | |
|---|---|
| Corn starch | 30 |
| Hydroxypropylcellulose | 6 |
| Magnesium stearate | 3 |
| | 200 mg/tablet |

Prescription Example 2 (Capsule)

Following components were mixed and charged into capsules in a conventional manner to prepare capsuled pharmaceutical agents.

| | |
|---|---|
| Compound of Example 5 | 200 (mg) |
| Crystalline cellulose | 50 |
| Silicic anhydride | 2 |
| Magnesium stearate | 3 |
| | 250 mg/capsule |

Prescription Example 3 (Granule)

Following compositions were mixed and packed in a conventional manner to prepare granular pharmaceutical agents.

| | |
|---|---|
| Compound of Example 8 | 500 (mg) |
| Lactose | 323 |
| Corn starch | 150 |
| Polyvinylpyrrolidone | 25 |
| Silicic anhydride | 2 |
| | 1000 mg/package |

Prescription Example 4 (Suppository)

Following components were mixed to prepare suppositories in a conventional manner.

| | |
|---|---|
| Compound of Example 11 | 300 (mg) |
| Witep-sol W-35 | 1700 |
| | 2000 mg/suppository |

Witep-sol W-35 has widely been known as a basic material for preparing a suppository and is available in various countries. This comprises a mixture of mono, di and tri-glycerides of saturated fatty acids from lauric acid ($C_{11}$) to stearic acid ($C_{17}$). Physico-chemical data of the specific Witepsol used are given as follows.

1. Melting point:
   33.5 - 35.5 ℃
2. Solidification point:
   27 - 32℃
3. Iodine colour index:
   below 3
4. Acid value:
   below 0.3
5. Saponification value:
   225 - 235
6. Iodine value:
   below 3
7. Hydroxyl value:
   40 - 50
8. Unsaponifiable matter:
   below 0.3
9. Mean molecular weight:
   about 615

CLAIMS:

1. A novel organosilicon compound represented by the general formula

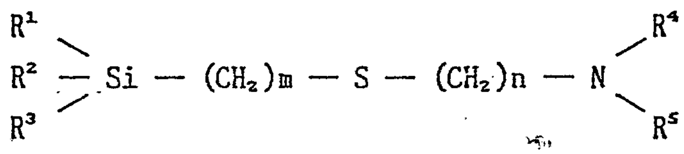

(I)

wherein $R^1$, $R^2$ and $R^3$ are each an alkyl, cycloalkyl, alkoxy, phenyl, substituted phenyl, alkylcarbonyloxy or tri-alkylsilyloxy group, $R^4$ and $R^5$ are each a hydrogen atom or an alkyl group, and m and n are each an integer, and non-toxic salts thereof.

2. A compound as claimed in Claim 1, wherein the alkyl group is a straight chain alkyl group having 1 to 10 carbon atoms.

3. A compound as claimed in Claim 1 or 2, wherein the substituents on a phenyl group are p-chloro, p-bromo or p-methoxy.

4. A non-toxic salt of a compound as claimedin Claim 1, 2 or 3 wherein the salt is a terephthalate or a maleate.

5. A process for the preparation of a compound as claimed in any of Claims 1 to 4, which comprises reacting a compound represented by the general formula.

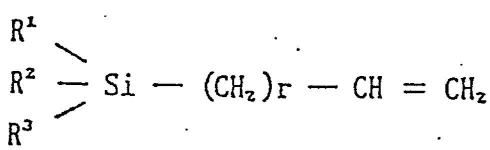

(II)

wherein $R^1$, $R^2$ and $R^3$ are as defined in Claim 1,2 or 3 and r is an integer,
with an ω-mercaptoalkylamine represented by the general formula

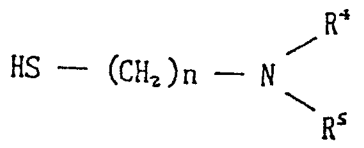

(III)

wherein $R^4$ and $R^5$ are as defined in Claim 1 or 2, and, if necessary converting the resulting compound into the non-toxic salt.

6. A process as claimed in Claim 5, wherein said reaction is carried out in a solvent under ultraviolet irradiation in the presence of a photosensitizive compound.

7. A 5-fluoroacil derivative of a compound as claimed in any of Claims 1 to 4.

8. An anti-tumor pharmaceutical composition comprising as an effective component a compound as claimed in any of Claims 1 to 4 or 7.

9. Use of a compound as claimed in any of Claims 1 to 4 or 7 or a composition as claimed in Claim 8 for the treatment of cancers and other tumors.